# EUROPÄISCHE PATENTANMELDUNG

(11) **EP 2 532 304 A1**
(43) Veröffentlichungstag der Anmeldung: **12.12.2012**
(21) Anmeldenummer: 12161643.7
(22) Anmeldetag: 28.03.2012
(51) Int. Cl.: A61B 5/107

(54) **Messvorrichtung zum Erfassen eines Bein- und Fußlängenmaßes zum Anmessen von Strümpfen, insbesondere medizinischen Kompressionsstrümpfen**

(30) Priorität: 06.06.2011 DE 102011104706
(71) Anmelder: medi GmbH & Co. KG, 95448 Bayreuth (DE)
(72) Erfinder: Doppel, Wiebke, 95326 Kulmbach (DE); Neugebauer, Beate, 95448 Bayreuth (DE)
(74) Vertreter: Blaumeier, Jörg

(57) **Zusammenfassung**

Messvorrichtung zum Erfassen eines Längenmaßes eines Beines zum Anmessen von Strümpfen, insbesondere medizinischen Kompressionsstrümpfen, umfassend eine Bodenplatte mit einer Fußaufnahmefläche und ein daran vertikal stehend angeordnetes Maßbrett, wobei das lösbar an der Bodenplatte (2) anbringbare Maßbrett (6) aus mehreren lösbar verbindbaren Brettabschnitten (6a, 6b, 6c, 6d) besteht.

## Beschreibung

Die Erfindung betrifft eine Messvorrichtung zum Erfassen eines Längenmaßes eines Beines zum Anmessen von Strümpfen, insbesondere medizinischen Kompressionsstrümpfen, umfassend eine Bodenplatte mit einer Fußaufnahmefläche und ein daran vertikal stehend angeordnetes Maßbrett.

Viele Menschen müssen vornehmlich aus phlebologischen Gründen besondere Strümpfe, vornehmlich medizinischen Kompressionsstrümpfe, tragen, die passgenau am Bein anliegen und einen definierten Druckverlauf (gemäß Gütesicherung RAL) aufbauen. Hierbei ist es erforderlich, die Strümpfe, die zum Teil auch Maßanfertigungen sind, möglichst exakt dem jeweiligen Umfang anzupassen. Hierzu dient eine Messvorrichtung der eingangs beschriebenen Art, mittels welcher es möglich ist, die Länge des Beines und Fußes bzw. des Bereichs sehr genau zu erfassen, längs welchem der Strumpf anliegen soll. Es kann sich hierbei um einen Bereich am Unterschenkel handeln, wenn nur ein kurzer Strumpf anzupassen ist, es kann aber auch die gesamte Beinlänge bis zur Taille sein, wenn es sich um einen langen Strumpf oder eine Strumpfhose handelt, die angepasst werden soll.

Eine bekannte Messvorrichtung besteht aus einer Bodenplatte mit einer Fußaufnahmefläche, auf die sich der Patient mit dem zu vermessenden Bein stellt. An der Rückseite der Bodenplatte ist ein vertikal zur Fußaufnahmefläche stehendes Maßbrett vorgesehen, das, wenn der Patient auf der Bodenplatte steht, sich hinter dem Bein erstreckt. Sowohl an dem Maßbrett als auch auf dem Fußteil ist eine Maßeinteilung, also ein Zentimetermaß mit entsprechender feiner Millimeterunterteilung auf der linken Seite, vorgesehen. Hierüber kann exakt die Länge des vom Strumpf zu belegenden Beinabschnitts erfasst werden. Auf der rechten Seite ist die Maßeinteilung in einzelne Barcodes verschlüsselt zur digitalen Übertragung in ein elektronisches Messsystem. Mittels eines flexiblen Maßbandes kann darüber hinaus zu entsprechenden Höhenpositionen auch der Umfang an definierten anatomischen Messpunkten ermittelt werden, so dass insgesamt unter Verwendung dieser Messvorrichtung ein sehr genaues Erfassen der Beinmaße möglich ist. Solche Messvorrichtungen werden beispielsweise in Arztpraxen, Kliniken oder Sanitätshäusern oder gegebenenfalls auch direkt vor Ort beim Patienten verwendet. Um eine solche Messvorrichtung auch einfach transportieren zu können, ist sie zusammenklappbar ausgeführt. Das heißt, dass die Bodenplatte und das Maßbrett über ein Scharnier schwenkbar miteinander verbunden sind. Um auch das Maßbrett in seiner Länge verkleinern zu können, ist auch dort wenigstens ein Scharnier vorgesehen, um es zusammenklappen zu können. Die aufgeschwenkten Teile sind in der Aufschwenkstellung über seitliche, üblicherweise aus Plastik bestehende Schieber, die zwei Teile verbinden, fixierbar.

Zwar lässt sich die Messvorrichtung über die Scharniere kleinformatig zusammenlegen, jedoch besteht beim Aufklappen und beim Zusammenlegen immer das Problem, dass Finger eingeklemmt werden können, dass sich also der Bediener verletzen kann. Auch werden die der Fixierung der Teile dienenden Schieber sehr leicht beschädigt, da sie bei unachtsamer Handhabung leicht abbrechen können.

Der Erfindung liegt damit das Problem zugrunde, eine Messvorrichtung anzugeben, die gegenüber bisher bekannten Messvorrichtungen verbessert ist.

Zur Lösung dieses Problems ist bei einer Messvorrichtung der eingangs genannten Art erfindungsgemäß vorgesehen, dass das lösbar an der Bodenplatte anbringbare Maßbrett aus mehreren lösbar verbindbaren Brettabschnitten besteht.

Die erfindungsgemäße Messvorrichtung ist in mehrere Teile vereinzelbar, nachdem sämtliche Einzelteile der Messvorrichtung lösbar miteinander verbindbar sind. Das heißt, dass das Maßbrett zum Transport von der Bodenplatte gelöst werden kann, wie auch das Maßbrett bestehend aus mehreren einzelnen Brettabschnitten in diese einzelne Brettabschnitte zerlegt werden kann. Zum Aufbau ist es also lediglich erforderlich, die Einzelteile miteinander zu verbinden, mithin also die Bodenplatte und den ersten Brettabschnitt zusammenzusetzen, und anschließend noch einen oder mehrere weitere Brettabschnitte zur Bildung des Maßbretts anzufügen. Eine Schwenkverbindung, bei der also die Teile zwangsläufig miteinander verbunden sind, ist erfindungsgemäß nicht gegeben, so dass hieraus resultierend die Verletzungsgefahr nicht mehr gegeben ist, da die Brettabschnitte untereinander sowie ein Brettabschnitt zur Bodenplatte bevorzugt über Steck- oder Schiebeverbindungen lösbar miteinander verbindbar sind, im Rahmen welcher also Elemente fest ineinander gesteckt oder ineinander geschoben werden. Es sind auch keine ein erneutes Zusammenklappen verhindernde Bauteile erforderlich, da bereits die Steck- oder Schiebeverbindungen eine gute Standfestigkeit verleihen. Darüber hinaus besteht die Möglichkeit, das Maßbrett auch nur so hoch "aufzubauen", wie es erforderlich ist. Besteht das Maßbrett beispielsweise aus maximal vier Brettabschnitten, so ist es ausreichend, wenn das Bein nur bis zum Knie zu vermessen ist, beispielsweise nur zwei Brettabschnitte zusammenzustecken oder zusammenzuschieben, was im Hinblick auf die Messaufgabe ausreichend ist. Ist es erforderlich, das gesamte Bein bis zur Taille zu vermessen, werden alle Brettabschnitte bis zur maximalen Maßbrettlänge miteinander verbunden.

Wie bereits beschrieben, erfolgt die lösbare Verbindung der Brettabschnitte untereinander respektive eines Brettabschnitts zur Bodenplatte über einfache Steck- oder Schiebeverbindungen. Eine Steckverbindung kann gemäß Weiterbildung dieses Erfindungsgedankens über einen oder mehrere am einen Brettabschnitt oder der Bodenplatte stirnkantenseitig vorspringende Steckabschnitte und eine oder mehrere am anderen Brettabschnitt oder der Bodenplatte stirnkantenseitig vorgesehene Steckabschnittaufnahmen realisiert werden. Die Steckabschnitte und die Steckabschnittaufnahmen greifen zweckmäßigerweise formschlüssig ineinander, so dass sich bereits dann eine hinreichende Stabilität des Steckaufbaus ergibt, wenn die Steckverbindungen ineinander greifen.

Eine Alternative zur Steckverbindung ist die Schiebeverbindung. Eine solche kann gemäß Weiterbildung dieser Erfindungsalternative über einen am einen Brettabschnitt oder der Bodenplatte stirnkantenseitig vorspringenden, federartigen Schiebeabschnitt und eine am anderen Brettabschnitt oder der Bodenplatte stirnkantenseitig vorgesehene Schiebenut realisiert sein. Bei dieser Erfindungsausgestaltung werden also zwei zu verbindende Teile quasi von der Seite her ineinandergeschoben, wozu wiederum an den zu verbindenden Stirnkanten entsprechende Verbindungsabschnitte in Form eines vorspringenden federartigen Schiebeabschnitts am einen Teil und einer entsprechenden Schiebenut am anderen Teil vorgesehen sind. Auch hier greifen Schiebeabschnitt und Schiebenut bevorzugt formschlüssig ineinander, um bereits nach dem Zusammenschieben eine hinreichende Stabilität zu gewährleisten. Auch diese Verbindungsmöglichkeit ist hinreichend einfach, so dass, ähnlich wie beim Zusammenstecken, die Messvorrichtung sehr einfach aufgebaut und zerlegt werden kann.

Die Steckverbindungen oder die Schiebeverbindungen können kodiert sein, so dass nur bestimmte Brettabschnitte miteinander bzw. mit der Bodenplatte verbindbar sind. Eine solche Kodierung kann dadurch realisiert sein, dass die Form und/oder Größe und/oder Position zumindest eines Teils der an den Brettabschnitten bzw. der Bodenplatte vorgesehenen Steckabschnitte und Steckaufnahmen oder Schiebeabschnitte und Schiebeaufnahmen unterschiedlich ist. Hierüber kann sichergestellt werden, dass nur bestimmte Teile miteinander zusammengesteckt oder ineinandergeschoben werden können und ein Vertauschen ausgeschlossen ist.

Wenngleich durch das Zusammenstecken respektive Zusammenschieben, insbesondere beim formschlüssigen Eingriff, bereits eine sehr gute Stabilität gegeben ist, sieht eine zweckmäßige Weiterbildung der Erfindung den Einsatz geeigneter Fixiermittel zum Fixieren zweier verbundener Brettabschnitte und/oder eines Brettabschnitts an der Bodenplatte vor. Diese Fixiermittel können beispielsweise eine Durchbrechung am einen Brettabschnitt oder der Bodenplatte durchsetzende, in ein Gewinde am zu verbindenden Brettabschnitt einzuschraubende Halteschrauben umfassen. Eine solche Halteschraube, beispielsweise eine mit einem Rändelkopf versehene Schraube, wird beispielsweise von der Längsstirnseite her in die Durchbrechung eingeschoben und in ein Gewinde, das beispielsweise an einem Steckabschnitt, der in die Steckaufnahme am die Durchbrechung aufweisenden Bauteil eingesetzt ist, eingeschraubt. Denkbar wäre aber natürlich auch, die Halteschraube von der Rückseite her in die dort befindliche Durchbrechung einzuführen und wiederum in ein Gewinde an einem Steckabschnitt einzuschrauben. In ähnlicher Weise kann die Befestigung bei einer Schiebeverbindung gegeben sein, wobei hier beispielsweise das Gewinde am federartigen Schiebeabschnitt vorgesehen wäre.

Alternativ zur Verwendung einer Halteschraube nebst Gewinde kann ein Fixiermittel auch eine Durchbrechung am einen Brettabschnitt oder der Bodenplatte durchsetzende, in eine Aufnahme zum verbindenden Brettabschnitt einzusteckende Haltestifte umfassen. Hier erfolgt also kein Verschrauben, sondern lediglich ein Einstecken des Haltestifts, so dass ein Gewinde nicht erforderlich ist. Bei dieser Erfindungsausgestaltung ist es möglich, die Haltestifte, die unverlierbar am jeweiligen Teil angeordnet sind, gegen jeweils ein Federelement bewegbar zu lagern, so dass sie beim Zusammenfügen zweier Brettabschnitte oder eines Brettabschnitts mit der Bodenplatte gegen das Federelement bewegt werden und bei Erreichen der Verbindungsstellung automatisch in die Aufnahmen einschnappen. Das heißt, dass der Anwender lediglich beispielsweise den oder die Steckabschnitte in die Steckaufnahmen einführen und die Teile zusammenschieben muss, wobei mit Erreichen der Endstellung der oder die Haltestifte aufgrund der Rückstellkraft der bis dahin vorgespannten Feder in die Aufnahmen einschnappen. Selbstverständlich ist der oder sind die Haltestifte aus dieser Einraststellung wieder gegen die Feder herausziehbar, um die Teile zu lösen.

Um die Haltestifte, die vor dem Zusammenstecken über die Feder quasi in die Schließstellung gedrängt werden, während des Zusammenschiebens nach außen zu bewegen, ist in Weiterbildung der Erfindung zum Bewegen eines federgelagerten Haltestifts gegen das Federelement eine Anlaufschräge am die Aufnahme aufweisenden Brettabschnitt vorgesehen. Befindet sich die Aufnahme beispielsweise an der Seite eines Steckabschnitts, so kann der Steckabschnitt an dieser Seite mit der Anlaufschräge versehen sein, die beim Zusammenstecken am Haltestift angreift und bei weiterer Zusammensteckbewegung den Stift gegen die Feder nach außen drängt. Mit Erreichen der Endstellung schnappt der federgelagerte Haltestift automatisch in die Aufnahme ein.

Ein Haltestift ist bevorzugt in seiner Lösestellung bei gespanntem Federelement fixierbar. Sollen zwei zusammengesteckte oder zusammengeschobene Teile gelöst werden, so ist der Haltestift aus seiner Einschnappstellung herauszuziehen. Um ihn zum Auseinanderziehen der Teile nicht dauernd halten zu müssen, ist es möglich, den Haltestift in der herausgezogenen Lösestellung zu fixieren, beispielsweise dadurch, dass er einfach um 90° verdreht wird. Dies ermöglicht es, zwei verbundene Teile auf einfache Weise zu lösen, auch dann, wenn, wie erfindungsgemäß bevorzugt vorgesehen ist, an beiden Seiten entsprechende Fixiermittel, in diesem Fall bevorzugt federgelagerte Haltestifte, vorgesehen sind.

Um zu vermeiden, dass die Fixiermittel seitlich weit hervorstehen, sind die Fixiermittel in Form der Halteschrauben oder Haltestifte in der Fixierstellung bevorzugt versenkt, bzw. liegen an der Messvorrichtung an, so dass sie von dieser nicht abstehen.

Die Fixiermittel sind, wie bereits beschrieben, bevorzugt an beiden Längsseiten der Bodenplatte oder der Brettabschnitte vorgesehen, was jedoch nicht zwingend erforderlich ist. Insbesondere bei Ausführung mit Schiebeverbindungen besteht die Möglichkeit, die Schiebenut und den Schiebeabschnitt nicht über die gesamte Länge zu ziehen, sondern vor dem jeweiligen Ende des Teils enden zu lassen, so dass ein Zusammenschieben nur von einer Seite her möglich ist. Da also ein Durchschieben nicht möglich ist, ist eine Fixierung nur an einer Stelle ausreichend.

Die Bodenplatte und die Brettabschnitte sind bevorzugt aus Kunststoff, insbesondere aus Polystyrol, was eine einfache Fertigung ermöglicht und Bruchsicherheit gewährleistet.

Dies bietet ferner die Möglichkeit, die Bodenplatte hinreichend dünn auszuführen, bevorzugt mit einer Dicke ≤ 1 cm. Dies ist dahingehend von Vorteil, als keine Ausgleichsplatte für das zweite Bein vorzusehen ist, die bei bis dato bekannten Messvorrichtungen, die relativ dicke, zumeist hohlkammerige Bodenplatten hatten, zu verwenden waren, um zu vermeiden, dass der Patient schief steht. Die dünne Ausführung der Bodenplatte als Vollmaterial ist auch dahingehend von Vorteil, als auch sehr schwere Patienten mit einem Gewicht über 100 kg ohne weiteres die Bodenplatte betreten können. Des Weiteren wurde das Maßbrett relativ breit (ca. 21 cm) ausgeführt, um auch größere Oberschenkelumfänge genau vermessen zu können.

Die Verwendung von Kunststoff als Material zur Ausbildung der Messvorrichtung ist dahingehend von Vorteil, als die Teile ohne weiteres bedruckt werden können, beispielsweise mit Siebdruck, auch ist eine Beschichtung mit Klarlack oder einer Überzugsfolie und dergleichen ohne weiteres möglich, um insbesondere das Maßbrett vor Verschmutzung, Verkratzen und Ähnlichem zu schützen, wie auch den Aufdruck abzudecken, so dass dieser beim Reinigen mit Desinfektionsmitteln nicht beschädigt werden kann. Bodenseitig sind bevorzugt Noppen und Ähnliches angeordnet, um die Bodenplatte rutschfest aufstellen zu können. Jedoch lässt die Messvorrichtung einen Einsatz sowohl bei stehendem Patienten zu, als auch bei liegendem Patienten, beispielsweise eines im Krankenbett liegenden Patienten. Denn infolge der vorgesehenen Teileverbindung durch festes Zusammenstecken oder Zusammenschieben wirken sich Unebenheiten beispielsweise im Krankenbett nicht nachteilig auf die Messung aus, da sich das Maßbrett hierüber nicht verformt.

Weitere Vorteile, Merkmale und Einzelheiten der Erfindung ergeben sich aus dem im Folgenden beschriebenen Ausführungsbeispiel sowie anhand der Zeichnungen. Dabei zeigen:
- Fig. 1: eine Perspektivansicht einer erfindungsgemäßen Messvorrichtung,
- Fig. 2: eine Explosionsansicht der Messvorrichtung aus Fig. 1,
- Fig. 3: eine vergrößerte Detailansicht zur Darstellung der Verbindung zweier Vorrichtungsteile einer ersten Ausführungsform,
- Fig. 4: eine vergrößerte Detailansicht zur Darstellung der Verbindung zweier Vorrichtungsteile einer zweiten Ausführungsform, und
- Fig. 5: eine vergrößerte Detailansicht zur Darstellung der Verbindung zweier Vorrichtungsteile einer dritten Ausführungsform.

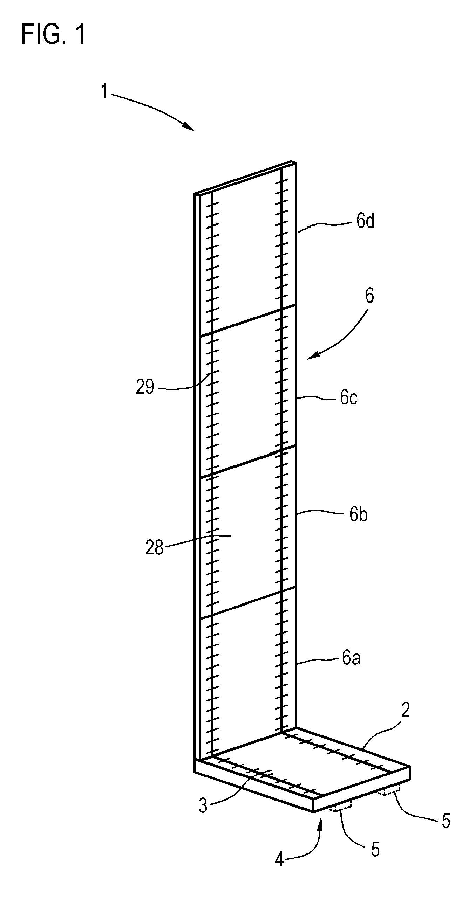

Fig. 1 zeigt eine erfindungsgemäße Messvorrichtung 1 zur Erfassung der Länge eines Beines und Fußes, was zum Anmessen von Strümpfen, vornehmlich medizinischen Kompressionsstrümpfen, erforderlich ist. Die Messvorrichtung 1 umfasst eine Bodenplatte 2 mit einer Fußaufnahmefläche 3, auf welcher der Patient während des Vermessens steht. An der Unterseite 4 der Bodenplatte 2 sind, hier nur gestrichelt angedeutet, entsprechende rutschsichere Auflagen 5 vorgesehen.

Die Messvorrichtung 1 umfasst ferner ein Maßbrett 6, das an dem hinteren Ende der Bodenplatte 2 lösbar befestigt ist. Das Maßbrett 6 selbst besteht im gezeigten Beispiel aus vier separaten Brettabschnitten 6a, 6b, 6c und 6d, die ebenfalls lösbar miteinander verbunden sind. Die lösbare Verbindung der genannten Bauteile ermöglicht es, die Messvorrichtung 1 auf einfache Weise durch Zusammenstecken oder Zusammenschieben, worauf nachfolgend noch eingegangen wird, aufzubauen sowie nach Gebrauch auseinanderzubauen und in eine kleinformatige Form, in der sie sich leicht transportieren lässt, zu zerlegen. An der Bodenplatte 2 und der zur Bodenplatte 2 weisenden Fläche 28 des Maßbretts 6 ist eine Längenskalierung 29 aufgebracht, beispielsweise aufgedruckt, die der Abnahme des Bein- und Fußlängenmaßes dient. Diese Längenskalierung 29 ist im gezeigten Beispiel nur lokal beispielsweise an den Seiten vorgesehen, sie kann selbstverständlich auch über die gesamte Breite aufgebracht sein.

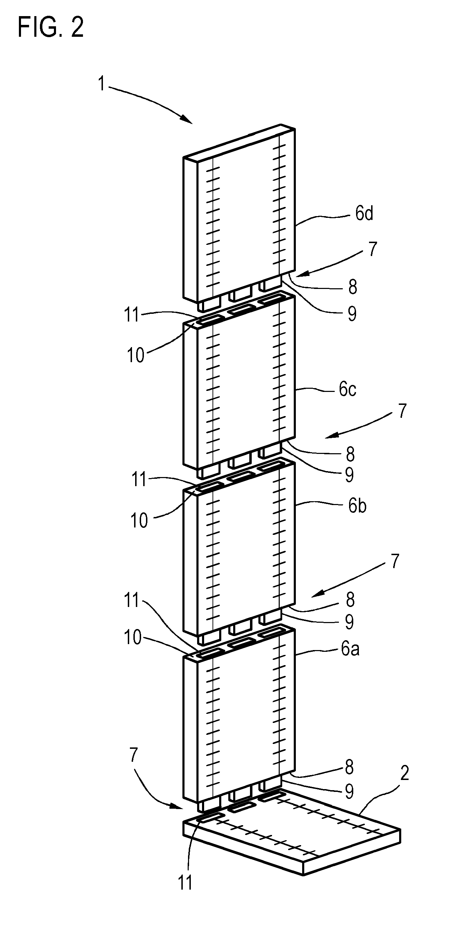

Fig. 2 zeigt als Explosionsansicht sämtliche einzelnen Teile der Messvorrichtung 1, nämlich das Bodenteil 2 sowie die vier Brettabschnitte 6a, 6b, 6c und 6d.

Um die Brettabschnitte 6a ― 6d untereinander lösbar verbinden zu können, wie auch den Brettabschnitt 6a an der Bodenplatte 2 lösbar anordnen zu können, sind entsprechende Steckverbindungen 7 realisiert, umfassend an den Stirnseiten 8 der Brettabschnitte 6a ― 6d vorspringende Steckabschnitte 9 sowie an der Bodenplatte 2 wie auch den gegenüberliegenden Stirnkanten 10 der Steckabschnitte 6a ― 6c ausgebildete Steckaufnahmen 11. Im gezeigten Beispiel sind die Steckabschnitte 9 und die Steckaufnahmen 11 rechteckig, sie sind also einander formangepasst, so dass eine formschlüssige Steckverbindung erreicht werden kann. Selbstverständlich sind auch andere Formen (oval, rund etc.) denkbar. Durch entsprechende unterschiedliche Positionierungen der Steckabschnitte 9 und der zugeordneten Steckaufnahmen 11 kann darüber hinaus eine Kodierung ausgebildet werden, die es ermöglicht, nur zusammengehörige Teile zusammenstecken zu können, das heißt, dass beispielsweise nur die Brettabschnitte 6a und 6b zusammengesteckt werden können, ein Einstecken des Brettabschnitts 6c in den Brettabschnitt 6a wäre nicht möglich, da die Steckabschnitte 9 des Steckabschnitts 6c nicht in die Steckaufnahmen 11 des Steckabschnitts 6a passen würden.

In jedem Fall kann folglich aus diesen gezeigten Teilen die Messvorrichtung 1 vor Ort sehr schnell durch einfaches Zusammenstecken aufgebaut und wieder zerlegt werden. Darüber hinaus bietet die Messvorrichtung den Vorteil, das Maßbrett 6 nur so hoch aufzubauen, wie es zum Vermessen tatsächlich erforderlich ist. Soll beispielsweise nur die Länge des Beins bis zum Knie bestimmt werden, um einen den Unterschenkel bedeckenden Strumpf anzupassen, sind nur die Brettabschnitte 6a und 6b nebst Bodenplatte 2 zusammenzustecken, soll das ganze Bein u. a. bis zur Taille vermessen werden, sind auch die beiden anderen Brettabschnitte 6c und 6d anzustecken.

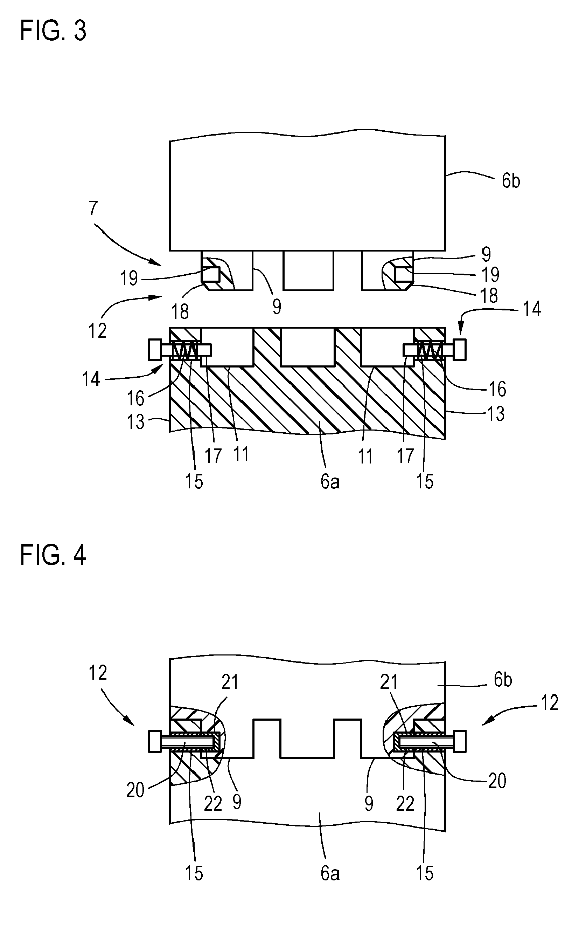

Fig. 3 zeigt eine Möglichkeit, wie eine Steckverbindung 7, die aufgrund der Formschluss-Passung von Haus aus bereits eine ausreichende Grundstabilität besitzt, fixiert werden kann, so dass die verbundenen Teile nicht ohne weiteres voneinander getrennt werden können. Hierzu sind Fixiermittel 12 vorgesehen, wobei im gezeigten Beispiel sowohl links als auch rechts entsprechende Fixiermittel angeordnet sind. Im gezeigten Beispiel umfassen die Fixiermittel am einen Bauteil, hier exemplarisch dem Brettabschnitt 6a, an den Längsstirnkanten 13 angeordnete Haltestifte 14, die dort in einer Durchbrechung 15, in der auch ein Federelement 16, hier eine Schraubenfeder, angeordnet ist, aufgenommen sind. Mit ihrem freien Ende 17 ragen die Federstifte in die benachbarte Steckaufnahme 11 ein. Die Federstifte 14 können aus der in Fig. 3 gezeigten Fixierstellung, in die sie automatisch über das Federelement 16 gebracht sind, gegen das Federelement 16 nach außen gezogen werden. Sie sind jedoch gegen ein Herausziehen gesichert. In dieser nach außen gezogenen Lösestellung können sie arretiert werden, beispielsweise indem der Federstift 14 etwas verdreht wird, so dass eine Arretierung greift, indem beispielsweise ein radialer Ansatz des Haltestifts 14 gegen einen Anschlag gedreht wird und dergleichen, so dass das Federelement 16 den jeweiligen Haltestift 14 nicht wieder zurückschieben kann. Diese Arretierung ist beim Auseinanderbauen zweckmäßig, als der Anwender die beiden Haltestifte links und rechts mit den Händen lösen kann, sie dann aber nicht mehr festhalten muss, sondern den Brettabschnitt 6b auf einfache Weise herausziehen kann.

Beim Zusammenstecken befinden sich die Haltestifte 14 nicht zuletzt aus Transportgründen in der in Fig. 3 gezeigten Fixierstellung. Es ist nun möglich, in dieser Stellung, ohne die Haltestifte 14 herausziehen zu müssen (was aber aufgrund der Möglichkeit zur Arretierung ohne weiteres möglich ist) den Brettabschnitt 6b anzustecken. Hierzu weisen die beiden seitlichen Steckabschnitte 9 schräg stehende Anlaufflächen 18 auf, sowie jeweils eine quer dazu angeordnete Aufnahme 19. Wird nun der Brettabschnitt 6b eingesteckt, so legen sich beim Hineinschieben der Steckabschnitte 9 in die Steckaufnahmen 11 die Anlaufflächen 18 an das freie Ende des jeweiligen Federstifts 14 und schieben diesen aufgrund der Schrägstellung bei zunehmendem Einschieben nach außen. Erreicht der Brettabschnitt 6b die Endstellung, liegen also die Steckabschnitte 9 tiefstmöglich in den Steckaufnahmen 11, so schnappen die Haltestifte 14 mit ihren freien Enden 17 automatisch über die sich dann entspannenden Federelemente 16 in die Aufnahme 19 ein und fixieren den eingesteckten Brettabschnitt 6b.

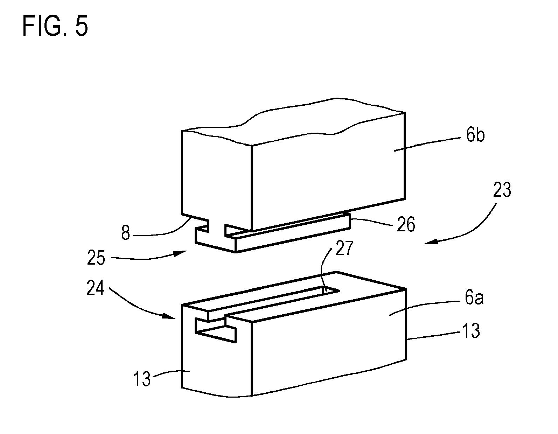

Eine weitere alternative Befestigungsmöglichkeit zeigt Fig. 4. Auch dort sind Fixiermittel 12 vorgesehen, bei denen es sich um Halteschrauben 20 handelt. Diese durchsetzen wiederum eine entsprechende Durchbrechung 15 am auch hier exemplarisch gezeigten Brettabschnitt 6a und werden zur Fixierung in eine Gewindehülse 21, die im jeweiligen Steckabschnitt 9 vorgesehen ist, mit ihrem Gewinde 22 eingeschraubt. Die Schrauben 20 (wie auch die Haltestifte 14) weisen einen entsprechend großen, manuell leicht zu greifenden Kopf auf, so dass das Ein- und Ausschrauben (respektive das Herausziehen im Falle der Haltestifte 14) entsprechend leicht vonstatten geht. In den jeweiligen Fixierstellungen liegen die Köpfe jedoch nahestmöglich an den Längsseiten des Maßbretts an, stehen also nicht ab. Fig. 5 zeigt schließlich ein weiteres Ausführungsbeispiel für eine Teileverbindung, hier in Form einer Schiebeverbindung 23. Diese Verbindungsart ist wiederum an allen zu verbindenden Teilen (also Bodenplatte 2 sowie sämtlichen Brettabschnitten 6a ― 6d) vorgesehen, exemplarisch sind hier wiederum nur die Brettabschnitte 6a und 6b gezeigt.

Am Brettabschnitt 6a ist eine Schiebenut 24 ausgebildet, die sich von der Längsstirnseite 13 am oberen Brettabschnittende horizontal erstreckt. Sie ist als beidseitig hinterschnittene T-Nut ausgeführt. Sie erstreckt sich nicht über die gesamte Brettabschnittsbreite, sondern nur bis kurz vor das Ende, um ein Einschieben nur von der linken Seite zu ermöglichen.

An der Unterseite 8 des Brettabschnitts 6b ist ein federartiger Schiebeabschnitt 25 ausgebildet, dessen Querschnittsform der Querschnittsform der Schiebenut 24 angepasst ist, mithin also ebenfalls T-förmig ist. Der Schiebeabschnitt 25 erstreckt sich ebenfalls nicht bis zum Ende, so dass über die Stirnkante 26 des Schiebeabschnitts 25 und das Ende 27 der Schiebenut 24 eine Anschlagbegrenzung realisiert ist.

Zum Verbinden der Brettabschnitte untereinander respektive des Brettabschnitts 6a mit der Bodenplatte 2, die über eine entsprechende Schiebenut 24 verfügt, sind die Teile lediglich von der Seite her ineinanderzuschieben. Aufgrund des formschlüssigen Eingriffs ergibt sich auch hier bereits bei Schließen der Schiebeverbindung eine hinreichende Stabilität. Zusätzlich können wiederum Fixiermittel 12 vorgesehen sein, beispielsweise eine von der rechten Längsseite 13 des Brettabschnitts 6a eingeschraubte Halteschraube 20, die in eine Gewindehülse 21, die an der Stirnseite 26 des Schiebeabschnitts 25 eingesetzt ist, eingeschraubt wird.

Die Bodenplatte 2 sowie die Steckabschnitte 6a ― 6d bestehen bevorzugt aus Kunststoff, vorzugsweise Polystyrol, weisen also von Haus aus gute Gleiteigenschaften auf, die ein einfaches Zusammenstecken oder Zusammenschieben ermöglichen.

## Patentansprüche

1. Messvorrichtung zum Erfassen eines Längenmaßes eines Beines zum Anmessen von Strümpfen, insbesondere medizinischen Kompressionsstrümpfen, umfassend eine Bodenplatte mit einer Fußaufnahmefläche und ein daran vertikal stehend angeordnetes Maßbrett, **dadurch gekennzeichnet, dass** das lösbar an der Bodenplatte (2) anbringbare Maßbrett (6) aus mehreren lösbar verbindbaren Brettabschnitten (6a, 6b, 6c, 6d) besteht.

2. Messvorrichtung nach Anspruch 1, **dadurch gekennzeichnet, dass** die Brettabschnitte (6a, 6b, 6c, 6d) untereinander und zur Bodenplatte (2) über Steck- oder Schiebeverbindungen (12, 23) lösbar miteinander verbindbar sind.

3. Messvorrichtung nach Anspruch 2, **dadurch gekennzeichnet, dass** eine Steckverbindung (12) über einen oder mehrere am einen Brettabschnitt (6a, 6b, 6c, 6d) oder der Bodenplatte (2) stirnkantenseitig vorspringende Steckabschnitte (9) und eine oder mehrere am anderen Brettabschnitt (6a, 6b, 6c, 6d) oder der Bodenplatte (2) stirnkantenseitig vorgesehene Steckabschnittaufnahmen (11) realisiert ist.

4. Messvorrichtung nach Anspruch 3, **dadurch gekennzeichnet, dass** die Steckabschnitte (9) und die Steckabschnittaufnahmen (11) formschlüssig ineinander greifen.

5. Messvorrichtung nach Anspruch 2, **dadurch gekennzeichnet, dass** eine Schiebeverbindung (23) über einen am einen Brettabschnitt (6a, 6b, 6c, 6d) oder der Bodenplatte (2) stirnkantenseitig vorspringenden, federartigen Schiebeabschnitt (25) und eine am anderen Brettabschnitt (6a, 6b, 6c, 6d) oder der Bodenplatte (2) stirnkantenseitig vorgesehene Schiebenut (24) realisiert ist.

6. Messvorrichtung nach Anspruch 5, **dadurch gekennzeichnet, dass** die Schiebeabschnitte (25) und die Schiebenuten (24) formschlüssig ineinander greifen.

7. Messvorrichtung nach einem der vorangehenden Ansprüche, **dadurch gekennzeichnet, dass** Fixiermittel (12) zum Fixieren zweier verbundener Brettabschnitte (6a, 6b, 6c, 6d) und/oder eines Brettabschnitts (6a, 6b, 6c, 6d) an der Bodenplatte (2) vorgesehen sind.

8. Messvorrichtung nach Anspruch 7, **dadurch gekennzeichnet, dass** die Fixiermittel (12) eine Durchbrechung (15) am einen Brettabschnitt (6a, 6b, 6c, 6d) oder der Bodenplatte (2) durchsetzende, in ein Gewinde (21) am zu verbindenden Brettabschnitt (6a, 6b, 6c, 6d) einzuschraubende Halteschrauben (20) umfassen.

9. Messvorrichtung nach Anspruch 7, **dadurch gekennzeichnet, dass** die Fixiermittel (12) eine Durchbrechung (15) am einen Brettabschnitt (6a, 6b, 6c, 6d) oder der Bodenplatte (2) durchsetzende, in eine Aufnahme (19) am zu verbindenden Brettabschnitt (6a, 6b, 6c, 6d) einzusteckende Haltestifte (14) umfassen.

10. Messvorrichtung nach Anspruch 9, **dadurch gekennzeichnet, dass** die Haltestifte (14) gegen ein Federelement (15) bewegbar gelagert sind, so dass sie beim Zusammenfügen zweier Brettabschnitte (6a, 6b, 6c, 6d) oder eines Brettabschnitts (6a, 6b, 6c, 6d) mit der Bodenplatte (2) gegen das Federelement (15) bewegt werden und bei Erreichen der Verbindungsstellung automatisch in die Aufnahmen (19) einschnappen.

11. Messvorrichtung nach Anspruch 10, **dadurch gekennzeichnet, dass** zum Bewegen eines federgelagerten Haltestifts (14) gegen das Federelement (15) eine Anlaufschräge (18) am die Aufnahme (19) aufweisenden Brettabschnitt (6a, 6b, 6c, 6d) vorgesehen ist.

12. Messvorrichtung nach Anspruch 10 oder 11, **dadurch gekennzeichnet, dass** die Haltestifte (14) in ihrer Lösestellung bei gespanntem Federelement (15) fixierbar sind.

13. Messvorrichtung nach einem der Ansprüche 8 bis 12, **dadurch gekennzeichnet, dass** die Halteschrauben (20) oder die Haltestifte (14) in der Fixierstellung versenkt oder am Maßbrett (6) oder der Bodenplatte (2) anliegend sind.

14. Messvorrichtung nach einem der Ansprüche 7 bis 13, **dadurch gekennzeichnet, dass** an beiden Längsseiten der Bodenplatte (2) oder der Brettabschnitte(6a, 6b, 6c, 6d) Fixiermittel (12) vorgesehen sind.

15. Messvorrichtung nach einem der vorangehenden Ansprüche, **dadurch gekennzeichnet, dass** die Bodenplatte (2) und die Brettabschnitte (6a, 6b, 6c, 6d) aus Kunststoff, insbesondere Polystyrol sind.

16. Messvorrichtung nach einem der vorangehenden Ansprüche, **dadurch gekennzeichnet, dass** die Bodenplatte (2) eine Dicke ≤ 1 cm aufweist.
